Europäisches Patentamt

**European Patent Office**

Office européen des brevets

(19)

(11) Publication number: **0 196 855**
**A2**

(12) **EUROPEAN PATENT APPLICATION**

(21) Application number: **86302172.1**

(22) Date of filing: **25.03.86**

(51) Int. Cl.⁴: **A 61 K 31/415**
**A 61 K 31/495**

(30) Priority: **29.03.85 US 717424**
**29.03.85 US 717423**

(43) Date of publication of application:
**08.10.86 Bulletin 86/41**

(84) Designated Contracting States:
**BE CH DE FR GB IT LI LU NL SE**

(71) Applicant: **PFIZER INC.**
**235 East 42nd Street**
**New York, N.Y. 10017(US)**

(72) Inventor: **Gibbs, David Lee**
**250, Mercer Street, Apt. A301**
**New York New York(US)**

(74) Representative: **Wood, David John et al,**
**Pfizer Limited Ramsgate Road**
**Sandwich Kent CT13 9NJ(GB)**

(54) **Tioconazole and related compounds for prevention of sexually transmitted diseases and control of herpetic infections.**

(57) The use of tioconazole, econazole, clotrimazole, ketoconazole, miconazole, butaconazole or a pharmaceutically
acceptable acid addition salt of any of these for the
manufacture of a medicament for the prevention of sexually
transmitted diseases or control of herpetic infections.

EP 0 196 855 A2

## TIOCONAZOLE AND RELATED COMPOUNDS FOR THE PREVENTION OF SEXUALLY TRANSMITTED DISEASES AND CONTROL OF HERPETIC INFECTIONS

This invention relates to the use of tioconazole and related imidazole antifungal agents for the prevention of sexually transmitted diseases (STD's) and control (chemoprophylactic or chemotherapeutic) of Herpes simplex virus (HSV), especially of genital herpes.

Tioconazole, 1-[2-{(2-chloro-3-thienyl)methoxy)-2-(2,4-dichlorophenyl}ethyl]-1H-imidazole, and related 1-aryl-2-(1-imidazolyl)alkyl ethers and thioethers are described in U.S. Patent 4,062,966, issued December 13, 1977, as antifungal agents for animals, including human, use. Said compounds may be administered in a variety of forms such as suppositories or pessaries or they may be applied topically in the form of, for example, creams, ointments or dusting powders.

U.S. Patent 4,247,552, issued January 27, 1981, discloses the spermatostatic and spermatocidal activity of tioconazole and related compounds, including econazole, miconazole, ketoconazole and clotrimazole, said compounds being administered intravaginally as creams, spray foams, sponges and the like to subjects clinically asymptomatic of vaginal microbial infection.

Control of STD's, including herpes infections, has been, and continues to be, a long sought goal in view of the major health problems posed by said diseases. Yoffe et al. Gynak, Rdsch. 23, suppl. 1, 37-41 (1983), report the effective short term treatment of vaginitis due to Trichomonas vaginalis by intravaginal administration of tioconazole. Jick et al. J.A.M.A. 248, 1619-1621 (1982) report that female users of non-antimicrobial spermicidal

0196855

agents such as nonoxynol, 9 [alpha (nonylphenyl) omega-hydroxypoly (oxy-1,2-ethanediyl)], had one-fourth the gonorrhea rate of non-users. The results were described as consistent with a protective effect of said non-antimicrobial vaginal spermicide against gonorrhea. Cates et al., J.A.M.A. 248, 1636-1637 (1982) summarize the results of Jick et al. (loc. cit.)and speculate that use of spermicides may be of value as vaginal chemical prophylactics against sexually transmitted diseases (STDs).

Schnell, Post-grad med. J. 1974, suppl. pp. 70-81 reports successful treatment of vaginal trichomoniasis by topical application of clotrimazole.

Jevons et al. Antimicrobial Agents Chemother. 15, 597 (1979) made known the in vitro activity of tioconazole against Gram positive Gardnerella vaginalis and Trichomonas vaginalis, causative agents of vaginitis.

U.S. Patent 4,315,001, issued February 9, 1982, discloses the treatment of Herpes simplex virus type 1 and type 2 infections in humans by administration of 2-deoxy-D-glucose either alone or in combination with an antifungal agent such as miconazole or miconazole nitrate.

A variety of aryloxyalkylpyrazoles which exhibit in vitro activity against HSV types 1 and 2 and in vivo activity against mouse genital HSV2 are described in U.S. Patents 4,171,365; 4,209,526; 4,232,161; 4,234,725 and 4,261,928.

It is interesting and surprising, to note that although tioconazole and the above-enumerated antifungal imidazoles have been known for several years to have spermatostatic and spermatocidal activity and have been used for the treatment of vaginitis, there have been no reports of their use or other topical antimicrobics to prevent STD's or control herpetic infections (HSV 1 and 2). If indeed any such uses occurred they were unintended and unappreciated; they were an unrecognized accident. Further, none of the reported studies or uses of said compounds were conducted with the intent of preventing STD's or controlling herpetic infections. Their purpose was treatment of existing fungal and STD infections; cure rather than prevention. There is, in fact, no known prior use of said compounds which can be considered to have consistently achieved prevention of STD's or control of herpetic infections.

Although condoms, diaphragms, creams, foams and gels having a barrier type effect reportedly prevent STDs, no antimicrobic chemoprophylactic methods are currently available for chemoprophylaxis of STDs. There thus exists a need for a chemoprophylactic method that is safe, efficacious and convenient to use.

Few drugs are known for the treatment of Herpes simplex type 1 infections and even fewer for the treatment of Herpes simplex type 2 infections. Both infections, but especially HSV2, genital Herpes, are sexually transmitted and pose a major health problem. There is, therefore, a pressing need for agents for the prophylactic and/or therapeutic treatment of persons subject to exposure to said infection and/or those suffering from said infection. The ability of a given drug to act both prophylactically and therapeutically is especially desirable.

It has now been found that tioconazole, miconazole, econazole, clotrimazole, ketoconazole and butaconazole are effective as topical vaginal dosage forms for the prevention (prophylaxis) of sexually transmitted diseases, i.e. those which, during periods of sexual activity by an individual, may be transmitted by pathogenic organisms

such as <u>Trichomonas</u> <u>vaginalis</u>, <u>Neisseria</u> <u>gonorrhoeae</u>, <u>Gardnerella</u> <u>vaginalis</u> and <u>Chlamydia</u> <u>trachomatis</u>.

It has also been found that these drugs are useful for the control of HSV1 and 2 (herpetic infections) on inanimate objects as well as in animals and humans subject to exposure thereto and/or infected thereby. They are highly valuable for the control of herpetic infections and especially for control of HSV2 in humans. The term "control" as used herein includes the chemoprophylaxis (or prevention) and chemotherapy (or treatment) of herpetic infections. When used for such purposes said agents are applied topically. They present a convenient, safe and efficacious precedure for the prevention and treatment of <u>Herpes</u> infections. Their use as regards <u>Herpes</u> is all the more surprising in view of the viral nature of said infection.

Also valuable for the same purposes and in the same way are pharmaceutically acceptable acid addition salts of tioconazole and the other compounds enumerated above. By said salts is meant those salts formed by said compounds with acids which form non-toxic acid addition salts therewith. Representative of such salts are the hydrochloride, hydrobromide, acetate, sulfate, maleate, gluconate and p-toluenesulfonate.

Thus the invention concerns the use of tioconazole, econazole, clotrimazole, ketoconazole, miconazole, butaconazole or a pharmaceutically acceptable acid addition salt thereof for the manufacture of a medicament for prevention of sexually transmitted diseases or control of herpetic infections.

The chemical names of the drugs described herein are presented below:

| Drug | Chemical Name |
|------|---------------|
| tioconazole | 1-[2-[(2-chloro-3-thienyl)methoxy]-2-(2,4-dichlorophenyl)ethyl]-1H-imidazole. |
| miconazole | 1-[2-(2,4-dichlorophenyl)-2-[2,4-dichlorophenyl)methoxy]ethyl]-1H-imidazole. |
| econazole | 1-[2-[(4-chlorophenyl)methoxy[-2-(2,4-dichlorophenyl)ethyl]-1H-imidazole. |
| clotrimazole | 1-[(2-chlorophenyl)diphenylmethyl]-1H-imidazole. |
| ketoconazole | 1-acetyl-4[4-[[2-(2,4-dichlorophenyl)-2-(1H-imidazol-1-ylmethyl)-1,3-dioxolan-4-yl]-methoxy]phenyl]piperazine. |
| butaconazole | 1-[4-(4-chlorophenyl)-2-[(2,6-dichlorophenyl)thio] butyl]-1H-imidazole. |

For the control of herpetic infections on inanimate objects or surfaces; i.e., basically as disinfectants, the herein described drugs and/or their pharmaceutically acceptable salts are generally applied in the form of dilute solutions or suspensions in organic or aqueous-organic solvents such as ethanol, acetone or dimethylsulfoxide and mixtures thereof with each other or water. They are applied to the locus on which control
of herpetic infections is desired in an anti-herpetic
controlling amount by immersing, spraying or swabbing
said locus. Concentrations of drug ranging from about
1% to about 10% by weight, or an equivalent weight of a
pharmaceutically acceptable salt thereof, are used. In
general, concentrations at the lower end of this range;
i.e., from about 1% to about 5%, are effective in
disinfecting inanimate surfaces.

For the control of herpetic infections in animals
and humans the herein-described drugs and/or their
pharmaceutically acceptable acid addition salts are
administered topically to the locus to be protected or
treated. For the control of genital herpes; i.e., HSV2
infections, said drugs and/or their pharmaceutically
acceptable acid addition salts are administered intravaginally preferably in admixture with a pharmaceutical
carrier. Said carrier is, of course, chosen with
regard to the intended route and method of administration.
In the present invention administration is accomplished
topically; i.e., to a definite place or locus, in this
instance, for example, the vagina, in the form of a
cream, ointment, foam, jelly, tablet, ovule or other
suitable composition which lends itself to a topical
vaginal dosage form. Creams and ointments are preferred
forms.

For the prevention of STDs tioconazole and related
antifungal imidazole agents enumerated above (drugs)
and/or their pharmaceutically acceptable acid addition
salts are administered intravaginally preferably in
admixture with a pharmaceutical carrier. Said carrier
is, of course, chosen with regard to the intended route
and method of administration. In the present invention
administration is accomplished topically; i.e., to a definite place, in this instance, for example, the vagina, in the form of a cream, ointment, foam, jelly, tablet, ovule or other suitable composition which lends itself to a topical vaginal dosage form. Creams and ointments are preferred forms.

Suitable creams for both indications comprise an aqueous emulsion of polyethylene glycols or liquid petrolatum and from 1% to 10% by weight of drug or an equivalent weight of a pharmaceutically acceptable acid addition salt thereof. Typical ointments comprise a white wax or white soft petrolatum base together with such stabilizers and preservatives as may be required and from 1% to 10% by weight of tioconazole.

Tioconazole or one of the above mentioned compounds can, of course, be administered alone. However, in keeping with standard pharmaceutical practice they are preferably administered in admixture with a pharmaceutical carrier to achieve more uniform distribution of the drug and to permit use of minimum drug concentrations sufficient to accomplish the intended purpose.

A favored topical vaginal dosage form is a cream as described above comprising from 1% to 2% by weight of one of said drugs. In each instance from about 1 to about 5 ml of said dosage form is applied intravaginally, desirably high in the vaginal vault. Greater amounts are generally avoided to minimize leakage.

The particular form of tioconazole or related compound enumerated above when used for control of herpetic infections in animals and humans is applied intravaginally prior to coitus. Alternatively, or even concurrently, it can be applied to consorts. For the chemotherapy of herpetic infections said drugs are administered topically to the site of infection and desirably to the immediate area surrounding said infection.

-8-

The particular form of tioconazole or related compound enumerated above when used for prevention of STDs is applied intravaginally prior to coitus. Alternatively, or even concurrently, it can be applied to consorts.

The activity of the compounds against HSV 1 and 2 is determined by exposing a suspension of the virus at approximately $10^6$ pfu/ml in buffer or buffer plus 5% serum to the drug, e.g., tioconazole, for various periods of time. Aliquots are removed at the end of each time period and titrated immediately on Vero cell tissue culture monolayers. Plaques are counted after 2-3 days incubation at 37°C.

The _in_ _vitro_ evaluation of the anti-herpetic activity of the compounds is performed by determining the minimum inhibitory concentration (m.i.c.) of the test compounds in a suitable medium at which growth of the particular micro-organism fails to occur. In practice a series of agar plates, each having the test compound incorporated at a particular concentration are inoculated with a standard culture of HSV1 or 2 and each plate is then incubated for 24 hours at 37°C. The plates are then examined for the presence or absence of growth of the virus and the appropriate m.i.c. value is noted.

In this manner, tioconazole has been found to be virucidal against HSV1 and HSV2 even after relatively short contact periods at levels of 50 mg/ml or higher. Against HSV1 tioconazole, at 100 mg/ml, exhibited >99.9% reduction in infectivity after 24 hours. Against HSV2, 50 mg/ml achieved 95% reduction in infectivity after 20 minutes, and >99.9% reduction at one hour and 24 hours after contact. A concentration of 50 mg of tioconazole /ml achieved 95% and >99.9% reduction in infectivity after 20 and 60 minutes contact, respectively. However, after 24 hours of exposure no reduction in infectivity was observed with tioconazole at 50 mg/ml.

The effectiveness of the herein-described drugs or a salt thereof in preventing STDs is assessed by determining the activity of said drug against the chosen STD pathogens by conventional agar or broth dilution methodology.

The growth inhibitory activity of the herein-described compounds against STD causing organisms is determined by standard methods. Activity against T. vaginalis was determined by an adaptation of the procedure of Squires et al. Brit. J. Vener. Dis. 38, 218 (1962). The procedure comprises culturing T. vaginalis isolates in liquid medium containing 20% inactivated horse serum. Serial two-fold dilutions of the compounds under test were incorporated in the medium and the minimum concentration required to kill the organisms (i.e. render them non-motile and granular) was recorded on microscopic observation following 72 hours incubation at 37°C. Clotrimazole was used as a comparative agent.

The susceptibility of G. vaginalis to said compounds was determined by the serial two-fold dilution method in peptone-starch-dextrose medium without blood or serum supplements. Results were read after incubation at 37°C. for 4 days.

The procedure of Ridgway et al., J. Antimicrob. Chemother. 2, 71 (1976) was used to evaluate the susceptibility of C. trachomatis to the herein-described compounds. A single strain of C. trachomatis was used, the laboratory-adapted lymphogranuloma venereum (LGV) strain, designated SA2f. The organism was propagated in McCoy cells (mouse L line) grown in Eagle's minimal

essential medium supplemented with foetal calf serum (10%), glutamine and vitamins. The test drug was incorporated into the medium at a range of concentrations determined by serial two-fold dilution and its effect on formulation of chlamydial intracellular inclusions was assessed after incubation at 35°C for 38-40 hours, Oxytetracycline served as control.

Representative formulations of tioconazole useful in this invention are presented below. The remaining agents disclosed herein are formulated and used in the same manner.

## TIOCONAZOLE VAGINAL TABLETS, 100 mg

| INGREDIENT | MG/TABLET |
|---|---|
| Tioconazole | 100.00 |
| Lactose | 644.00 |
| Corn starch | 372.00 |
| Magnesium stearate | 10.80 |
| Sodium lauryl sulphate | 1.20 |
| | 1,128.00 |

<div align="center">

**Tioconazole Cream**

**1% and 2%**

</div>

|  | g/Kg | |
|---|---|---|
| Ingredient | 1.0% | 2.0% |
| Tioconazole | 10.00 | 20.00 |
| White Soft Paraffin | 20.00 | 20.00 |
| Liquid Paraffin | 25.00 | 25.00 |
| Stearyl Alcohol | 55.00 | 55.00 |
| Stearic Acid | 45.00 | 45.00 |
| Cetomacrogol 1000* | 10.00 | 10.00 |
| Benzyl Alcohol | 10.00 | 10.00 |
| Propylene Glycol | 100.00 | 100.00 |
| Purified Water | 725.00 | 715.00 |
|  | 1000.00 | 1000.00 |

*Identified in the Merck Index, 10th Edition, entry no. 7449, published by Merck & Co., Inc. 1983, as a polyethylene glycol fatty alcohol ether of formula $CH_3(CH_2)_m(OCH_2CH_2)_nOH$ where m may be 15-17 and n may be 20-24.

<div align="center">

**Tioconazole Ointment**

**6.50%**

</div>

| Ingredient | g/Kg |
|---|---|
| Tioconazole | 65.0 |
| Veegum F | 53.9 |
| White Soft Paraffin | 881.1 |
|  | 1000.0 |

-12-

## Tioconazole Vaginal Ovules, 100 mg

| Component | Grade | Mg/Ovule |
|---|---|---|
| Tioconazole | (91050) | 100.00 |
| Glycine (milled) | USP | 1250.00 |
| Lecithin (soya) | (93-CI-080) | 20.00 |
| Hydrogenated Vegetable Fat | (93-CI-081) | 160.00 |
| Beeswax | BP | 40.00 |
| Polysorbate 80* | EP | 30.00 |
| Liquid Paraffin, Liquid | PPC (1963) | 653.00 |
| | | 2253.00 |

*Sorbitan: mono-9-octadecenoate poly (oxy-1,2-ethanediyl), available from Atlas Chemical Industries, Inc. of Wilmington, Delaware.

-13-

## CLAIMS

1. The use of tioconazole, econazole, clotrimazole, ketoconazole, miconazole, butaconazole or a pharmaceutically acceptable acid addition salt of any one of these compounds for the manufacture of a medicament for the prevention of sexually transmitted diseases or control of Herpetic infections.

2. The use of tioconazole according to claim 1.

3. The use as claimed in claim 1 & 2 for the prevention of sexually transmitted diseases.

4. The use as claimed in claim 1 & 2 for the control of herpetic infections.

5. The use as claimed in claim 4 for the control of genital Herpes.